# DEMANDE DE BREVET EUROPEEN

(11) **EP 4 403 137 A1**
(43) Date de publication de la demande: **24.07.2024**
(21) Numéro de dépôt: 23197327.2
(22) Date de dépôt: 14.09.2023
(51) Int. Cl.: A61F 2/24, A61M 60/896, A61M 60/894, A61M 60/196, A61M 60/268, A61M 60/859

(54) **SYSTÈME DE FIXATION D'UNE VALVE CARDIAQUE SUR UNE PROTHÈSE CARDIAQUE ET PROTHÈSE CARDIAQUE POURVUE D'UN TEL SYSTÈME DE FIXATION**

(30) Priorité: 17.01.2023 FR 2300413
(71) Demandeur: CARMAT, 78140 Vélizy Villacoublay (FR)
(72) Inventeur: ESTORD, Sébastien, 78350 Jouy en Josas (FR); PICARD, Jean-François, 78955 Carrières sous Poissy (FR)
(74) Mandataire: Gevers & Orès

(57) **Abrégé**

- Système de fixation d'une valve cardiaque sur une prothèse cardiaque et prothèse cardiaque pourvue d'un tel système de fixation.
- Le système (1) a pour fonction de fixer une valve cardiaque (3) sur une prothèse cardiaque (2) destinée à être implantée dans la cavité péricardique d'un patient et à remplacer les ventricules gauche et droit naturels du patient, ce système (1) comportant une bague mâle (10) pourvue de picots (11) et une pièce coopérante (12) pourvue d'ouvertures (13), la bague mâle (10) ou la pièce coopérante (12) étant destinée à être solidarisée de la prothèse cardiaque (2), les picots (11) et les ouvertures (13) étant configurées pour que lesdits picots (11) traversent une collerette (9) de la valve cardiaque (3) et pénètrent dans les ouvertures (13) dans une position de fixation, ce système (1) pouvant être utilisé pour fixer des valves cardiaques (3) de réalisations variées.

## Description

### DOMAINE TECHNIQUE

La présente invention concerne un système de fixation d'une valve cardiaque sur une prothèse cardiaque, ladite prothèse cardiaque étant implantable dans la cavité péricardique d'un patient et étant apte à remplacer les ventricules gauche et droit naturels dudit patient après leur ablation. La présente invention concerne également une prothèse cardiaque pourvue d'un tel système de fixation.

### ÉTAT DE LA TECHNIQUE

Une prothèse cardiaque totalement implantable, telle que décrite par exemple dans les brevets FR-2 784 585 et FR-2 902 345, a pour fonction de remplacer les ventricules du patient en conservant les oreillettes. Pour ce faire, elle comporte un corps de prothèse rigide dans lequel sont notamment agencés des ventricules artificiels. Pour l'implantation de cette prothèse cardiaque, c'est-à-dire une prothèse de coeur artificiel, dans la cavité péricardique du patient, il est nécessaire de créer des interfaces entre le réseau vasculaire (oreillettes et artères) du patient et la prothèse cardiaque en respectant l'anatomie du patient.

On sait que le coeur naturel comprend quatre valves cardiaques qui séparent les différentes cavités et assurent une bonne circulation du sang entre elles, à savoir deux valves cardiaques (valve mitrale et valve tricuspide) situées entre les oreillettes et les ventricules et deux valves cardiaques (valve aortique et valve pulmonaire) situées entre les ventricules et les artères. Une prothèse cardiaque, totalement implantable, qui est destinée à remplacer le coeur naturel, doit donc intégrer des valves cardiaques (artificielles) afin de recréer cette connexion spécifique avec les oreillettes et les artères.

Afin de pouvoir utiliser à cet effet des valves cardiaques usuelles, utilisées dans le cadre de chirurgies de remplacement valvulaire, la prothèse cardiaque totalement implantable doit intégrer une interface spécifique. Une valve cardiaque usuelle comprend, en général, trois feuillets qui s'ouvrent et se ferment sous l'action de la pression, trois jambages auxquels sont fixés les feuillets et une base qui permet au chirurgien de suturer la valve. La base peut être plane ou ondulée (en forme de vague).

De telles valves cardiaques peuvent être utilisées pour l'admission ou l'éjection du sang dans la prothèse cardiaque.

Généralement, pour l'éjection, la valve cardiaque est de type à base plane et elle repose avec sa base plane dans un canal de la prothèse cardiaque, les jambages étant orientés vers l'extérieur des ventricules. Un conduit en tissu est agencé dans le canal de la prothèse cardiaque au-dessus de la valve cardiaque. Un système de verrouillage verrouille l'ensemble sur la prothèse cardiaque, le conduit en tissu étant ensuite suturé sur l'artère du patient par le chirurgien.

Ce mode de fixation utilisé à l'éjection n'est pas complètement satisfaisant et présente les inconvénients suivants :
- une distance courte entre le point le plus haut de la valve cardiaque et le point de suture du conduit en tissu sur l'artère (artère pulmonaire ou aorte) du patient, qui peut gêner le chirurgien pour réaliser la suture ;
- la nécessité de comprimer l'anneau de la valve cardiaque et le conduit en tissu dans le canal de la prothèse cardiaque pour assurer l'étanchéité au sang de la liaison, ce qui demande un effort d'insertion important de la part du chirurgien. Cette opération est rendue encore plus complexe en raison de l'absence de point de préhension de la prothèse cardiaque et du risque de torsion de l'agrafe servant au verrouillage ;
- les matériaux et les dimensions de l'ensemble formé de la valve cardiaque et du conduit en tissu, qui est installé dans le canal de la prothèse cardiaque, ne sont pas les plus performants en terme d'étanchéité ; et
- ce mode de fixation ne permet pas d'utiliser des valves cardiaques avec une base non plane, par exemple en forme de vague.

### EXPOSÉ DE L'INVENTION

La présente invention a pour objet de remédier à au moins certains des inconvénients précités, dans le but de simplifier et d'améliorer l'opération de fixation de valves cardiaques artificielles sur une prothèse cardiaque (destinée à être implantée dans la cavité péricardique d'un patient afin de remplacer les ventricules gauche et droit naturels du patient), et de pouvoir utiliser des valves cardiaques de réalisations variées et notamment des valves cardiaques avec des bases non planes. Pour ce faire, elle concerne un système de fixation destiné à la fixation d'une valve cardiaque (artificielle) sur une prothèse cardiaque.

Selon l'invention, ledit système de fixation comporte une bague mâle pourvue de picots, et une pièce coopérante pourvue d'ouvertures, ladite bague mâle ou ladite pièce coopérante étant destinée à être solidarisée de la prothèse cardiaque, et les picots et les ouvertures sont configurées pour que lesdits picots traversent une collerette de la valve cardiaque et pénètrent dans lesdites ouvertures dans une position dite de fixation.

Ainsi, grâce à l'invention, la bague mâle et la pièce coopérante qui sont destinées à maintenir la valve cardiaque au niveau de la collerette, peuvent être adaptées à cette collerette, à savoir à la base de la valve cardiaque, comme précisé ci-dessous. Le système de fixation peut ainsi être adapté à des valves cardiaques de réalisations variées et notamment des valves cardiaques avec des bases non planes, et ceci aussi bien pour des valves cardiaques utilisées pour l'éjection que pour des valves cardiaques utilisées pour l'admission.

Comme également précisé ci-dessous, ledit système de fixation présente beaucoup d'autres avantages, et notamment les avantages suivants :
- il permet de ne pas allonger le temps opératoire au bloc (par exemple, pas de suture avec les pièces d'interface) ;
- il permet un montage aisé par le chirurgien ;
- il permet de garantir l'absence de fuite de sang de l'intérieur des ventricules vers l'extérieur de la prothèse cardiaque ;
- il permet de conserver la valve cardiaque dans son emballage et de l'ouvrir pour son implantation uniquement au bloc opératoire ; et
- il permet de garder le sinus de Valsalva pour la partie admission de la prothèse cardiaque.

En fonction du mode de réalisation envisagé, et comme précisé ci-dessous, l'un des deux éléments (la bague mâle ou la pièce coopérante) est destiné à être rendu solidaire de la prothèse cardiaque.

Avantageusement, la bague mâle et la pièce coopérante présentent, chacune, une face dite de contact, ces faces de contact étant destinées à enserrer au moins la collerette de la valve cardiaque dans la position de fixation, et lesdites faces de contact présentent des formes adaptées à la forme de la collerette de la valve cardiaque.

Dans un premier mode de réalisation, les picots et les ouvertures sont configurées pour que les picots soient sensiblement rectilignes dans la position de fixation.

En outre, dans un second mode de réalisation, les picots et les ouvertures sont configurés pour que les picots soient courbés (ou cintrés) dans la position de fixation, par exemple coudés ou avec une forme courbe. Cette déformation permet de renforcer le maintien des picots dans les ouvertures et ainsi le maintien entre les différentes pièces liées ensemble.

Les picots peuvent présenter un diamètre constant. Toutefois, dans une réalisation particulière, les picots présentent un diamètre qui se réduit vers une extrémité libre, ce qui permet de faciliter le maintien des picots dans les ouvertures.

Dans un mode de réalisation préféré, la bague mâle qui comprend au moins un support annulaire et les picots solidaires du support annulaire est une pièce monobloc, ce qui permet de faciliter sa réalisation. Dans une variante de réalisation, il est toutefois également envisageable que les picots soient rapportés sur le support annulaire.

Par ailleurs, dans un premier mode de réalisation, la pièce coopérante est une bague femelle. Dans ce premier mode de réalisation, en fonction de la variante de réalisation envisagée, l'une de ces deux bagues (la bague mâle ou la bague femelle) est destinée à être fixée sur la prothèse cardiaque.

Avantageusement, la bague femelle comprend une face opposée à la face de contact, qui présente une forme complémentaire d'une forme d'une face dite de réception de la prothèse cardiaque.

En outre, dans un second mode de réalisation, la pièce coopérante correspond à une partie de la prothèse cardiaque. De préférence, ladite partie de la prothèse cardiaque comprend un élément tubulaire qui est pourvu, sur sa périphérie interne, d'un rebord destiné à recevoir la collerette de la valve cardiaque.

Par ailleurs, dans un mode de réalisation particulier, pour une valve cardiaque destinée à l'éjection, les picots et les ouvertures sont configurées pour que lesdits picots traversent également une collerette d'un conduit (destiné à être suturé sur une artère) dans la position de fixation.

En outre, dans un mode de réalisation particulier, le système de fixation comporte, de plus, au moins l'un des éléments suivants :
- un joint torique ;
- une agrafe de fixation ;
- une bague de maintien.

La présente invention concerne également une prothèse cardiaque implantable dans la cavité péricardique d'un patient, ladite prothèse cardiaque étant apte à remplacer les ventricules gauche et droit naturels dudit patient et comprenant au moins une valve cardiaque.

Selon l'invention, la prothèse cardiaque comporte au moins un système de fixation tel que celui décrit ci-dessus, pour fixer la valve cardiaque sur la prothèse cardiaque.

Dans un mode de réalisation préféré, la prothèse cardiaque comporte deux, trois ou (de préférence) quatre valves cardiaques dont chacune est fixée sur la prothèse cardiaque, à l'aide d'un système de fixation tel que celui décrit ci-dessus.

### BRÈVE DESCRIPTION DES FIGURES

Les figures annexées feront bien comprendre comment l'invention peut être réalisée. Sur ces figures, des références identiques désignent des éléments semblables.
La figure 1 est une vue éclatée, en perspective, d'un premier mode de réalisation d'un système de fixation.
La figure 2 est une vue, en perspective, du système de fixation de la figure 1, dans une position de montage.
La figure 3 est une vue, en perspective, du système de fixation de la figure 1, dans une position de fixation.
La figure 4 est une vue en coupe du système de fixation dans la position de fixation de la figure 3.
La figure 5 est une vue éclatée, en perspective, d'une première variante d'un second mode de réalisation d'un système de fixation
La figure 6 est une vue en coupe du système de fixation de la figure 5, dans une position de fixation.
La figure 7 est une vue éclatée, en perspective, d'une seconde variante d'un second mode de réalisation d'un système de fixation.

### DESCRIPTION DÉTAILLÉE

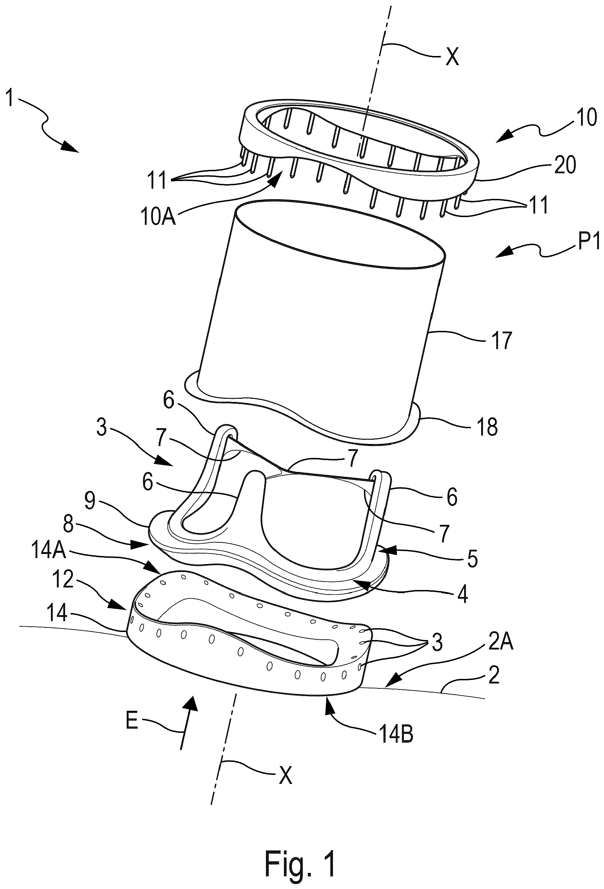

Le système de fixation 1 illustrant l'invention et représenté schématiquement dans différents modes de réalisation particuliers sur les figures 1 à 7 a pour fonction de réaliser la fixation d'une valve cardiaque artificielle (destinée à remplacer une valve cardiaque naturelle d'un patient) sur une prothèse cardiaque.

Cette prothèse cardiaque 2 (représentée très partiellement et très schématiquement sur les figures 1 et 5 à 7) est implantable dans la cavité thoracique et péricardique d'un patient et est apte à remplacer ses ventricules gauche et droit naturels, après leur ablation. Pour ce faire, la prothèse cardiaque 2 comporte un corps de prothèse rigide (non représenté), dans lequel sont notamment agencés des ventricules gauche et droit artificiels destinés à remplacer les ventricules gauche et droit naturels du patient.

La prothèse cardiaque 2 comporte tous les moyens nécessaires à son fonctionnement. En particulier, elle peut comporter au moins certaines des caractéristiques présentées dans les brevets FR-2 784 585 et FR-2 902 345 précités. La prothèse cardiaque 2, et notamment les éléments agencés dans le corps de prothèse, ne sont pas décrits davantage dans la description suivante.

La prothèse cardiaque 2 destinée à remplacer le coeur naturel du patient comprend, comme le coeur naturel, quatre valves cardiaques, à savoir deux valves cardiaques (valve mitrale et valve tricuspide) prévues entre les oreillettes et les ventricules artificiels de la prothèse cardiaque 2, pour l'admission, et deux valves cardiaques (valve aortique et valve pulmonaire) prévues entre les ventricules artificiels de la prothèse cardiaque 2 et les artères (aorte et artère pulmonaire) du patient, pour l'éjection.

Dans une mise en oeuvre préférée, au moins une valve cardiaque 3, mais de préférence l'ensemble des quatre valves cardiaques 3 correspondent, chacune, à une valve cardiaque usuelle, utilisée dans le cadre de chirurgies de remplacement valvulaire.

Dans un mode de réalisation particulier, chaque valve cardiaque 3 (artificielle) comprend, comme représenté sur la figure 1, une base 4 de forme annulaire, qui est pourvue, sur une face 5 aval, de trois jambages 6. La valve cardiaque 3 comprend également trois feuillets 7. Chaque feuillet 7 est fixé à deux jambages 6 adjacents et à la base 4. Les trois feuillets 7 de la valve cardiaque 3 sont configurées pour s'ouvrir et se fermer sous l'action de la pression dans la partie amont. Dans la présente description, les termes amont et aval sont définis selon le sens de circulation du sang à travers la valve cardiaque 3, comme illustré par une flèche E sur les figures 1, 2, 5 et 7.

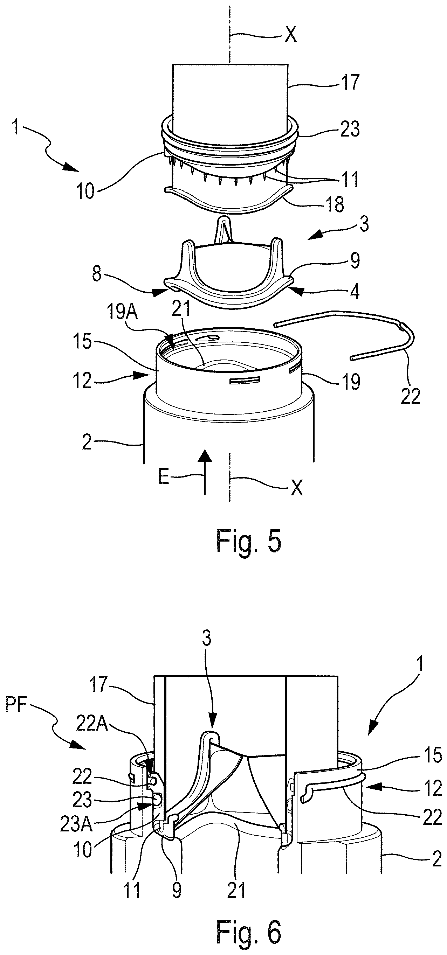

De plus, la base 4 présente une face 8 amont, opposée à la face 5 aval, et elle comprend une collerette 9 qui l'entoure (autour de toute sa périphérie extérieure). La face 8 amont (ainsi que la collerette 9) peuvent présenter des formes variées. Dans un premier mode de réalisation (non représenté), la face 8 amont et la collerette 9 sont planes. En outre, dans un second mode de réalisation particulier, comme représenté notamment sur les figures 1 et 5, la face 8 amont et la collerette 9 sont courbes ou ondulées, par exemple en forme générale de vague.

Le système de fixation 1 a pour fonction de fixer une telle valve cardiaque 3, quelle que soit la forme de sa base 4, sur la prothèse cardiaque 2.

Pour ce faire, le système de fixation 1 comporte, comme représenté notamment sur la figue 1, une bague mâle 10 de forme annulaire, qui est pourvue sur une face 10A (longitudinale) de picots 11. Les picots 11 correspondent à des éléments (notamment des tubes pleins) allongés faisant saillie sur la face 10A.

Dans un mode de réalisation préféré, la bague mâle 10 (qui comprend notamment un support annulaire 20 et les picots 11 solidaires du support annulaire 20) est une pièce monobloc, ce qui permet de faciliter sa réalisation. Cette pièce monobloc est, par exemple, réalisée en titane. Il est également envisageable dans une variante de réalisation, non représentée, que les picots représentent des éléments individuels qui sont rapportés (par exemple soudés ou collés) sur le support annulaire.

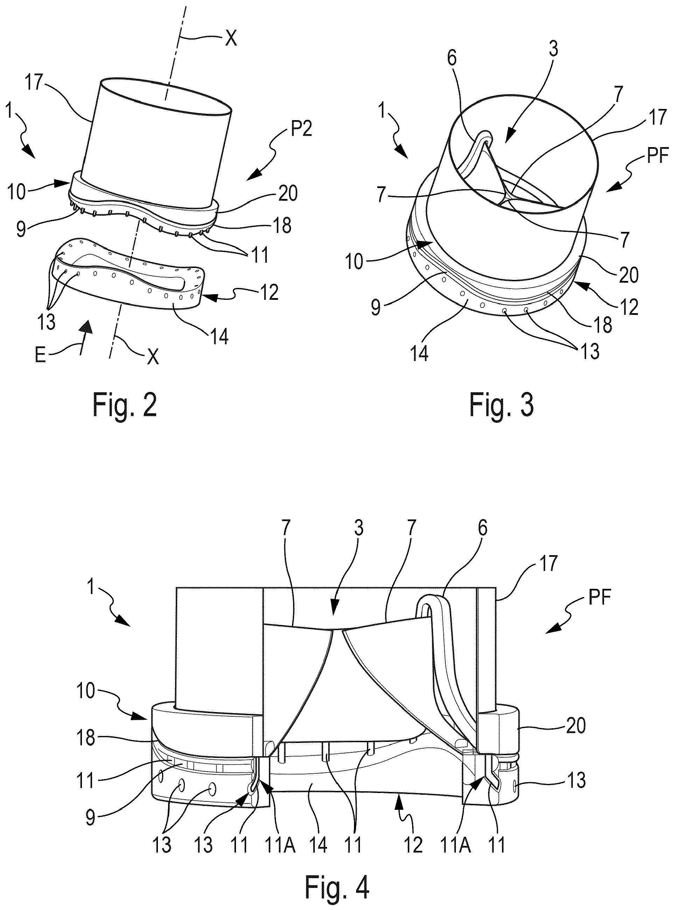

Le système de fixation 1 comporte également une pièce coopérante 12 destinée à coopérer avec la bague mâle 10. Cette pièce coopérante 12 est pourvue d'ouvertures 13. Chacune des ouvertures 13 est destinée à recevoir un picot 11 de la bague mâle 10 dans une position de fixation PF (figures 3, 4 et 6). La pièce coopérante 12 comprend ainsi au moins autant d'ouvertures 13 que la bague mâle 10 comprend de picots 11, et les picots 11 et les ouvertures 13 sont formés et agencés pour que chacun des picots 11 puisse être inséré dans une ouverture 13.

Dans le cadre de la présente invention, on considère que la position de fixation PF représente la position dans laquelle la valve cardiaque 3 est intégrée dans la prothèse cardiaque 2 et fixée, dans sa position finale, sur la prothèse cardiaque 2 à l'aide du système de fixation 1.

L'un des éléments suivants : la bague mâle 10, la pièce coopérante 12, est destiné à être solidarisé de la prothèse cardiaque 2. Différents modes de réalisation sont envisagés pour cette solidarisation, comme précisé ci-dessous.

De plus, les picots 11 et les ouvertures 13 sont configurés, comme également précisé ci-dessous, de telle manière que les picots 11 traversent la collerette 9 (formée par exemple d'une bande de silicone et d'un tissu PTFE poreux) de la valve cardiaque 3, avant de pénétrer dans les ouvertures 13, dans la position de fixation PF.

Par conséquent, dans la position de fixation PF, la bague mâle 10 et la pièce coopérante 12 enserrent la collerette 9 de la valve cardiaque 3, comme représenté sur les figures 3, 4 et 6.

De plus, comme précisé ci-dessous, les picots 11 sont maintenus par contact latéral dans les ouvertures 13, ce qui permet de solidariser la bague mâle 10 à la pièce coopérante 12 et les maintenir ensemble, et ceci éventuellement (dans un mode de réalisation particulier) avec l'aide d'un élément supplémentaire (par exemple une agrafe). Cette solidarisation permet de maintenir la valve cardiaque 3 par l'intermédiaire de la collerette 9 et ainsi de fixer la valve cardiaque 3 à la pièce coopérante 12 et, par conséquent, à la prothèse cardiaque 2 (à laquelle est solidarisée la pièce coopérante 12).

La bague mâle 10 et la pièce coopérante 12 présentent, chacune, une face dite de contact 10A et 14A (figure 1). Les faces de contact 10A et 14A sont destinées à enserrer la collerette 9 dans la position de fixation PF. Ces faces de contact 10A et 14A présentent des formes adaptées à la forme de la collerette 9 et donc à la forme de la base 4.

Le système de fixation 1 peut ainsi être adapté à la forme de la collerette 9 et de la base 4 de la valve cardiaque 3. Il peut notamment être adapté à une valve cardiaque 3 présentant une base 4 ondulée. Ainsi, le système de fixation 1 permet la fixation de tout type de valve cardiaque 3 usuelle et notamment une valve cardiaque 3 à base 4 non plane, pour son intégration dans la prothèse cardiaque 2.

De plus, le système de fixation 1 peut être utilisé soit pour la fixation d'une valve cardiaque 3 destinée à l'éjection du sang hors de la prothèse cardiaque 2, soit pour la fixation d'une valve cardiaque 3 destinée à l'admission du sang dans la prothèse cardiaque 2, comme précisé ci-dessus.

Le système de fixation 1, tel que décrit plus en détail ci-dessous, représente une interface qui permet de satisfaire les contraintes suivantes, liées à son utilisation dans une prothèse cardiaque 2 implantable :
- elle est biocompatible ;
- elle est implantable pour une longue durée ;
- elle est nettoyable et apte à être stérilisée ;
- elle garantit l'étanchéité avec la prothèse cardiaque 2 ; et
- elle est pourvue d'un mode de fixation ergonomique et rapide (qui permet de limiter la durée de la circulation extracorporelle).

Le système de fixation 1 peut être réalisé de différentes manières. Il peut, notamment, être réalisé de deux manières différentes en fonction du type de solidarisation de la pièce coopérante 12 ou de la bague mâle 10 à la prothèse cardiaque 2.

Dans un premier mode de réalisation représenté sur les figures 1 à 4, la pièce coopérante 12 est une bague femelle 14, c'est-à-dire une pièce indépendante destinée à être liée à la prothèse cardiaque 2. La bague femelle 14 représente une contreforme de la bague mâle 10. Dans ce premier mode de réalisation, l'une des deux bagues (la bague mâle 10 ou la bague femelle 14) est fixée sur la prothèse cardiaque 2.

Ainsi, dans une première variante (préférée), la bague femelle 14 est fixée de façon usuelle sur la prothèse cardiaque 2, et dans une seconde variante, la bague mâle 10 est fixée de façon usuelle sur la prothèse cardiaque 2.

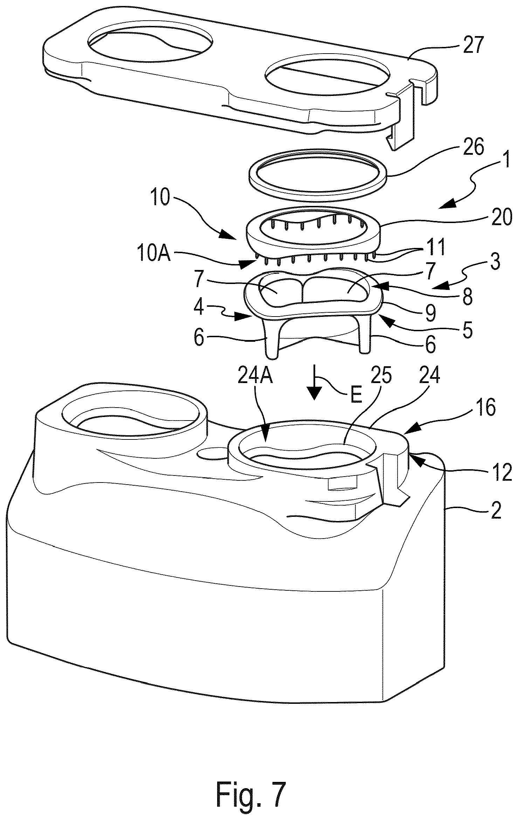

En outre, dans un second mode de réalisation, la pièce coopérante 12 correspond à une partie 15, 16 de la prothèse cardiaque 2, comme représenté selon deux variantes différentes sur les figures 5 à 7, à savoir une première variante sur les figures 5 et 6 pour la fixation d'une valve cardiaque 3 destinée à l'éjection et une seconde variante sur la figure 7 pour la fixation d'une valve cardiaque 3 destinée à l'admission.

Dans le premier mode de réalisation représenté sur les figures 1 à 4, la pièce coopérante 12 est donc une bague femelle 14, par exemple en titane, initialement indépendante et séparée de la prothèse cardiaque 2, qui présente une face 14B amont de forme particulière, destinée à venir au contact de la prothèse cardiaque 2. Cette forme particulière est complémentaire de la forme d'une face 2A aval dite de réception (représentée très schématiquement sur la figure 1) de la prothèse cardiaque 2.

Ce premier mode de réalisation comprenant la bague femelle 14, représenté sur les figures 1 à 4, concerne un système de fixation 1 pour la fixation d'une valve cardiaque 3 destinée à l'éjection.

La figure 1 montre les différents éléments dans une vue éclatée. Dans ce mode de réalisation (destiné donc à l'éjection), le système de fixation 1 a pour fonction de lier à la prothèse cardiaque 2, en plus de la valve cardiaque 3, également un conduit 17. Ce conduit 17 est destiné à amener du sang éjecté de la prothèse cardiaque 2 (dans le sens de la flèche E) au travers de la valve cardiaque 3 à une artère (aorte ou artère pulmonaire) à laquelle le conduit 17 est suturé de façon usuelle.

Le conduit 17 réalisé en tissu comprend également une collerette 18 à son extrémité amont. Cette collerette 18, par exemple en polyester, présente une forme similaire à la collerette 9 de la valve cardiaque 3. Dans ce mode de réalisation particulier, les picots 11 de la bague mâle 10 sont destinés à traverser (ou transpercer) également la collerette 18 du conduit 17.

Pour pouvoir être traversées ou transpercées par les picots 11, la collerette 9 de la valve cardiaque 3, et le cas échéant, la collerette 18 du conduit 17 doivent soit comprendre des trous (de taille appropriée), soit (de préférence) être réalisées en un matériau permettant aux picots 11 de les transpercer.

Les figures 1, 2 et 3 montrent trois étapes successives lors de la fixation de la valve cardiaque 3 et du conduit 17 sur la prothèse cardiaque 2.

Plus précisément :
- la figure 1 illustre une position initiale P1 dans laquelle les différents éléments ne sont pas liés entre eux (ou assemblées) ;
- la figure 2 illustre une position intermédiaire P2 lors de l'assemblage, dans laquelle la bague mâle 10 a été assemblée à la collerette 18 du conduit 17 et à la collerette 9 de la valve cardiaque 3. Les picots 11 de la bague 10 traversent ces collerettes 9 et 18 ; et
- la figure 3 illustre la position de fixation PF. Cette position de fixation PF est également représentée (en coupe) sur la figure 4.

Dans ce mode de réalisation, les picots 11 de la bague mâle 10 transpercent donc la collerette 18 du conduit 17 ainsi que la collerette 9 de la valve cardiaque 3 avant d'être insérés dans les ouvertures 13 de la bague femelle 14.

Dans un mode de réalisation préféré, les picots 11 de la bague mâle 10 sont agencés parallèlement à l'axe longitudinal X-X de la valve cardiaque 3 et du conduit 17 lorsque la bague mâle 10 est positionnée coaxialement par rapport à cet axe longitudinal, X-X, comme représenté sur les figures 1 à 7.

Dans le premier mode de réalisation, la bague femelle 14 comprend des ouvertures 13 de forme cylindrique, c'est-à-dire rectilignes, qui sont inclinées par rapport à l'axe longitudinal X-X, c'est-à-dire leur orientation présente un angle non nul par rapport à cet axe longitudinal X-X comme représenté sur la figure 4,

Ainsi, soumis à une pression lors de l'assemblage et contraints par l'inclinaison des ouvertures 13, les picots 11 sont amenés à se cintrer (ou se courber), comme représenté sur la figure 4. Par conséquent, en pénétrant dans les ouvertures 13, les picots 11 changent de direction et ils présentent un coude 11A (figure 4) dans leur position de fixation PF. Dans une variante de réalisation (non représentée), ils peuvent présenter une autre forme courbe, différente de cette forme avec un coude.

Cette déformation mécanique permet à l'assemblage des quatre pièces (bague mâle 10, collerette 18 du conduit 17 d'éjection, collerette 9 de la valve cardiaque 3 et bague femelle 14) de devenir un ensemble monobloc.

La bague femelle 14 peut être adaptée au contenu et à la forme de la prothèse cardiaque 2 et facilite ainsi l'intégration de la valve cardiaque 3. La bague mâle 10 et la bague femelle 14 peuvent être adaptées pour permettre la fixation, quel que soit le mode de réalisation considéré, de tout type de valve cardiaque 3 sans avoir à modifier le design de la prothèse cardiaque 2.

Le système de fixation 1 peut être réalisé selon différentes tailles et/ou formes afin, notamment, de s'adapter à des valves cardiaques 3 chirurgicales variées.

Pour le premier mode de réalisation, on a uniquement représenté une réalisation (préférée) destinée à l'éjection. Il est également envisageable de prévoir un tel mode de réalisation à bagues mâle et femelle coopérantes pour l'admission.

Par ailleurs, afin de s'adapter au mieux à la prothèse cardiaque 2 et de minimiser l'encombrement du système de fixation 1, le second mode de réalisation prévoit de supprimer la bague femelle en adaptant la forme d'une partie de la prothèse cardiaque 2 à une contreforme de la bague mâle 10.

Dans ce second mode de réalisation, la pièce coopérante 12 correspond donc à une partie 15, 16 de la prothèse cardiaque 2. Ce second mode de réalisation peut également être utilisé pour la fixation d'une valve cardiaque 3 destinée à l'éjection ou pour la fixation d'une valve cardiaque 3 destinée à l'admission.

Dans la première variante de ce second mode de réalisation, représentée sur les figures 5 et 6, qui est également destinée comme le premier mode de réalisation des figures 1 à 4 à fixer une valve cardiaque 3 utilisée pour l'éjection, la pièce coopérante 12 correspond à une partie 15 de la prothèse cardiaque 2. Cette partie 15 de la prothèse cardiaque 2 comprend, dans cet exemple, un élément (ou tronçon) tubulaire 19.

Dans ce mode de réalisation, l'élément tubulaire 19 est pourvu tout autour de sa périphérie interne 19A d'un rebord 21 de forme adaptée à la forme de la base 4 de la valve cardiaque 3. La forme de vague de la valve cardiaque 3 est ainsi intégrée dans le corps de la prothèse cardiaque 2. La face 8 amont de la base 4 de la valve cardiaque 3 repose sur ce rebord 21 dans la position de fixation PF, comme représenté sur la figure 6.

Dans cette variante des figures 5 et 6, les picots 11 de la bague mâle 10 ont toujours pour fonction de transpercer la collerette 18 du conduit 17 et la collerette 9 de la valve cardiaque 3, avant de se loger dans la pièce coopérante 1, préalablement usinée dans le corps de la prothèse cardiaque 2.

Dans cette réalisation particulière, les ouvertures 13 sont parallèles à l'axe longitudinal X-X du système de fixation 1, de sorte que, dans la position de fixation PF, les picots 11 ne sont pas cintrés (ou courbés) mais restent rectilignes et parallèles à l'axe longitudinal X-X, comme visible sur la figure 6.

Dans cette variante de réalisation, pour augmenter le maintien de la bague mâle 10, on peut prévoir une agrafe 22 qui est réalisée de façon usuelle pour retenir ensemble les pièces assemblées. Pour ce faire, on prévoit de préférence une gorge 22A usuelle (figure 6), destinée à recevoir l'agrafe 22.

De plus, dans l'exemple des figures 5 et 6, on a également prévu un joint torique 23 au niveau d'une gorge 23A (figure 6) de la bague mâle 10, qui est destiné à assurer l'étanchéité des pièces assemblées par rapport au flux sanguin.

Dans le cadre de la présente invention, quel que soit le mode de réalisation, les picots 11 peuvent présenter un diamètre constant. Toutefois, dans une réalisation particulière, les picots 11 présentent un diamètre qui se réduit vers leur extrémité libre (c'est-à-dire leur extrémité opposée à celle qui est liée à la face 10A de la bague mâle 10), ce qui permet de faciliter le montage et de renforcer le maintien, lorsqu'ils sont insérés dans des ouvertures 13 cylindriques (c'est-à-dire de diamètre constant).

Cette première variante du second mode de réalisation comprenant la partie 15 comme contreforme de la bague mâle 10 permet une bonne adaptation à la prothèse cardiaque et permet de minimiser l'encombrement du système de fixation 1.

Cette variante qui nécessite de modifier le design de la prothèse cardiaque 2 si la forme (par exemple) le modèle de la valve cardiaque 3 change, offre toutefois l'avantage de réduire la hauteur de dépassement des jambages de la valve cardiaque 3.

Par ailleurs, la seconde variante du second mode de réalisation, représentée sur la figure 7, est destinée à fixer une valve cardiaque 3 utilisée pour l'admission. Dans ce cas, les extrémités libres des feuillets 7 de la valve cardiaque 3 sont dirigées vers la prothèse cardiaque 2, c'est-à-dire en sens opposé au mode de réalisation des figures 5 et 6.

Dans cette seconde variante, la pièce coopérante 12 correspond à une partie 16 de la prothèse cardiaque 2. Cette partie 16 de la prothèse cardiaque 2 comprend, dans cet exemple, un élément (ou tronçon) tubulaire 24.

Dans ce mode de réalisation, l'élément tubulaire 24 est pourvu tout autour de sa périphérie interne 24A d'un rebord 25 de forme adaptée à la forme de la base 4 de la valve cardiaque 3. La forme de vague de la valve cardiaque 3 est ainsi intégrée dans le corps de la prothèse cardiaque 2. La face 8 amont de la base 4 de la valve cardiaque 3 repose sur ce rebord 25 dans la position de fixation (non représentée).

De plus, la bague mâle 10 qui est en contact avec le sang, est rendue hémocompatible.

Dans ce mode de réalisation, destiné à l'admission, une bague de maintien 26, de préférence un anneau métallique, est utilisée pour maintenir la bague mâle 10 avec les picots 11 et la valve cardiaque 3 dans la prothèse cardiaque 2 le temps de venir l'installer sur une lunette 27 métallique usuelle, déjà en place dans le thorax du patient, lors de l'implantation de la prothèse cardiaque 2. A titre d'illustration, les brevets FR-2 902 343 et FR-2 902 344 décrivent un système de lunettes coopérantes pour les admissions d'une prothèse cardiaque.

Dans une variante de réalisation (non représentée), le support annulaire 20 de la bague mâle 10 peut être dimensionné et conformé pour réaliser ce maintien (avant l'installation sur la lunette métallique), au lieu de la bague de maintien 26.

Il est également envisageable de prévoir, pour l'admission, un système de fixation selon le premier mode de réalisation à bagues mâle et femelle coopérantes. Dans une telle réalisation (non représentée), on remplace la partie 16 de l'exemple de la figure 7, par une bague femelle destinée à être fixée sur la prothèse cardiaque.

Par ailleurs, dans le cadre de la présente invention, en fonction du mode de réalisation particulier considéré, le système de fixation 1 peut comporter l'un ou plusieurs des éléments suivants :
- un joint torique tel que, par exemple, le joint torique 23 représenté sur les figures 5 et 6 ;
- une agrafe de fixation telle que, par exemple, l'agrafe 22 représentée sur les figures 5 et 6 ; et
- une bague de maintien telle que, par exemple, la bague de maintien 26 représentée sur la figure 7.

Par ailleurs, quel que soit le mode de réalisation considéré, pour répondre à la contrainte d'hémocompatibilité, la ou les pièces du système de fixation 1 en contact avec le sang (principalement la bague femelle 14 dans le mode de réalisation des figures 1 à 4) peuvent être recouvertes d'une prothèse tricotée (armure de tricotage de fil polyester). Dans une application à la bague femelle, de préférence, ce tissu en polyester est découpé au plus proche de la forme de la bague, il est enroulé autour de la bague et il est suturé pour être maintenu.

En variante, pour rendre hémocompatible les pièces en contact avec le sang, on peut prévoir d'autres solutions telles que, par exemple, le recouvrement de la surface en contact avec le sang avec du ePTFE, du microbillage de titane, ...

Quel que soit le mode de réalisation considéré, le procédé de mise en place du système de fixation 1 comprend au moins une opération consistant à faire transpercer la collerette 9 de la valve cardiaque 3 (et le cas échéant la collerette 18 du conduit 17) par les picots 11 de la bague mâle 10 et à insérer lesdits picots 11 dans les ouvertures 13 coopérantes de la pièce coopérante 12. En fonction du mode de réalisation considéré, ledit procédé comprend d'autres étapes mises en oeuvre avant et après cette opération.

Le système de fixation 1, tel que décrit ci-dessus, se substitue aux opérations de suture, tout en offrant une armature rigide à la valve cardiaque 3, qui facilite son intégration dans le design de la prothèse cardiaque 2.

Le système de fixation 1, présente de nombreux avantages. Tout d'abord, il permet d'obtenir les avantages principaux suivants :
- réduire la distance entre le point le plus haut de la valve cardiaque 3 et le point le plus haut de l'interface au niveau du conduit d'éjection de la prothèse cardiaque 2 ;
- permettre l'utilisation d'une valve cardiaque 3 dont la base 4 n'est pas plane mais intègre une forme de vague ;
- simplifier la préparation et l'implantation de la prothèse cardiaque 2.

En outre, le système de fixation 1 présente également les avantages suivants :
- il permet de ne pas allonger le temps opératoire au bloc (par exemple, pas de suture avec les pièces d'interface) ;
- il permet un montage aisé par le chirurgien ;
- il permet de garantir l'absence de fuite de sang de l'intérieur des ventricules vers l'extérieur de la prothèse cardiaque 2 ;
- il permet de conserver la valve cardiaque 3 dans son emballage et l'ouvrir pour son implantation uniquement au bloc opératoire ; et
- il permet de garder le sinus de Valsalva pour la partie admission de la prothèse cardiaque 2.

## Revendications

1. Système de fixation destiné à la fixation d'une valve cardiaque sur une prothèse cardiaque, ladite prothèse cardiaque (2) étant destinée à être implantée dans la cavité péricardique d'un patient et à remplacer les ventricules gauche et droit naturels du patient,
**caractérisé en ce qu'**il comporte une bague mâle (10) pourvue de picots (11), et une pièce coopérante (12) pourvue d'ouvertures (13), ladite bague mâle (10) ou ladite pièce coopérante (12) étant destinée à être solidarisée de la prothèse cardiaque (2), et **en ce que** les picots (11) et les ouvertures (13) sont configurées pour que lesdits picots (11) traversent une collerette (9) de la valve cardiaque (3) et pénètrent dans lesdites ouvertures (13) dans une position dite de fixation (PF).

2. Système selon la revendication 1,
**caractérisé en ce que** la bague mâle (10) et la pièce coopérante (12) présentent, chacune, une face dite de contact, ces faces de contact (1 0A, 14A, 21, 25) étant destinées à enserrer au moins la collerette (9) de la valve cardiaque (3) dans la position de fixation (PF), et **en ce que** lesdites faces de contact (10A, 14A, 21, 25) présentent des formes adaptées à la forme de la collerette (9) de la valve cardiaque (3).

3. Système selon l'une des revendications 1 et 2,
**caractérisé en ce que** les picots (11) et les ouvertures (13) sont configurées pour que les picots (11) soient sensiblement rectilignes dans la position de fixation (PF).

4. Système selon l'une des revendications 1 et 2,
**caractérisé en ce que** les picots (11) et les ouvertures (13) sont configurés pour que les picots (13) soient courbés dans la position de fixation (PF).

5. Système selon l'une quelconque des revendications précédentes, **caractérisé en ce que** les picots (11) présentent un diamètre qui se réduit vers une extrémité libre.

6. Système selon l'une quelconque des revendications précédentes, **caractérisé en ce que** la bague mâle (10) qui comprend au moins un support annulaire (20) et les picots (11) solidaires du support annulaire (20) est une pièce monobloc.

7. Système selon l'une quelconque des revendications 1 à 6,
**caractérisé en ce que** la pièce coopérante (12) est une bague femelle (14).

8. Système selon les revendications 2 et 7,
**caractérisé en ce que** la bague femelle (14) comprend une face (14B), opposée à la face de contact (14A), qui présente une forme complémentaire d'une forme d'une face dite de réception (2A) de la prothèse cardiaque (2).

9. Système selon l'une quelconque des revendications 1 à 6,
**caractérisé en ce que** la pièce coopérante (12) correspond à une partie (15, 16) de la prothèse cardiaque (2).

10. Système selon la revendication 9,
**caractérisé en ce que** ladite partie (15, 16) de la prothèse cardiaque (2) comprend un élément tubulaire (19, 24) qui est pourvu sur sa périphérie interne (19A, 24A) d'un rebord (21, 25) destiné à recevoir la collerette (9) de la valve cardiaque (3).

11. Système selon l'une quelconque des revendications précédentes, **caractérisé en ce que**, pour une valve cardiaque (3) destinée à l'éjection, les picots (11) et les ouvertures (13) sont configurées pour que lesdits picots (11) traversent également une collerette (18) d'un conduit (17) dans la position de fixation (PF).

12. Système selon l'une quelconque des revendications précédentes, **caractérisé en ce qu'**il comporte, de plus, au moins l'un des éléments suivants :
- un joint torique (23) ;
- une agrafe de fixation (22) ;
- une bague de maintien (26).

13. Prothèse cardiaque implantable dans la cavité péricardique d'un patient, ladite prothèse cardiaque (2) étant apte à remplacer les ventricules gauche et droit naturels dudit patient et comprenant au moins une valve cardiaque (3),
**caractérisée en ce qu'**elle comporte au moins un système de fixation (1) selon l'une quelconque des revendications 1 à 12 pour fixer la valve cardiaque (3) sur la prothèse cardiaque (2).

14. Prothèse cardiaque selon la revendication 13,
**caractérisée en ce qu'**elle comporte deux, trois ou quatre valves cardiaques (3) dont chacune est fixée sur la prothèse cardiaque (2), à l'aide d'un système de fixation (1) selon l'une quelconque des revendications 1 à 12.
